# EUROPEAN PATENT SPECIFICATION

(11) **EP 3 480 601 B1**
(45) Date of publication and mention of the grant of the patent: **30.03.2022**
(21) Application number: 16906845.9
(22) Date of filing: 06.07.2016
(51) Int. Cl.: G01N 33/533, G01N 33/74, G01N 21/76, G01N 33/58, G01N 33/543

(54) **INHIBIN B CHEMILUMINESCENT IMMUNOASSAY KIT AND PREPARATION METHOD THEREFOR**
INHIBIN-B-CHEMILUMINESZENZ-IMMUNOASSAY-KIT UND HERSTELLUNGSVERFAHREN DAFÜR
KIT DE DOSAGE IMMUNOLOGIQUE CHIMIOLUMINESCENT DE L'INHIBINE B ET PROCÉDÉ DE PRÉPARATION S'Y RAPPORTANT

(30) Priority: 30.06.2016 CN 201610506673
(43) Date of publication of application: 08.05.2019
(73) Proprietor: Shenzhen Yhlo Biotech Co., Ltd, 518116 Shenzhen (CN)
(72) Inventor: QIAN, Chungen, 518116 Shenzhen (CN); XIA, Fuzhen, 518116 Shenzhen (CN); WANG, Gang, 518116 Shenzhen (CN); ZHU, Liang, 518116 Shenzhen (CN); LIU, Meiting, 518116 Shenzhen (CN)
(74) Representative: Mewburn Ellis LLP
(86) International application number: PCT/CN2016/088817
(87) International publication number: WO 2018/000446

(56) References cited:
- CN-A- 102 072 957
- CN-A- 102 816 754
- CN-A- 103 149 350
- CN-A- 103 954 779
- CN-A- 103 954 779
- CN-A- 104 090 106
- CN-A- 104 849 469
- CN-A- 105 527 444
- MCCONNELL D S ET AL: "DEVELOPMENT OF A TWO-SITE SOLID-PHASE IMMUNOCHEMILUMINESCENT ASSAY FOR MEASUREMENT OF DIMERIC INHIBIN-A IN HUMAN SERUM AND OTHER BIOLOGICAL FLUIDS", CLINICAL CHEMISTRY, P.B. HOEBER, vol. 42, no. 8, 1 January 1996 (1996-01-01), pages 1159-1166, XP002045969, ISSN: 0009-9147
- KALRA B ET AL: "Development of a second generation Inhibin B ELISA", JOURNAL OF IMMUNOLOGICAL METHODS, ELSEVIER SCIENCE PUBLISHERS B.V.,AMSTERDAM, NL, vol. 362, no. 1-2, 31 October 2010 (2010-10-31), pages 22-31, XP027509586, ISSN: 0022-1759, DOI: 10.1016/J.JIM.2010.08.002 [retrieved on 2010-08-21]

## Description

### TECHNICAL FIELD

The present disclosure relates to the field of in vitro detection, and more particularly relates to an inhibin B chemiluminescence immunoassay kit and a preparation method thereof.

### BACKGROUND

Inhibin (INH) is a heterodimeric protein hormone secreted by female ovarian granulosa cells and male testicular support cells, belonging to the transforming growth factor beta family, mainly including INHA and INH-B (consisting of α and βA or βB subunits, respectively). Inhibin selectively inhibits the secretion of follicle stimulating hormone (FSH) and also has a local paracrine action on the gonads.

Inhibin B is a testis-derived glycoprotein hormone. Serum inhibin B levels in adult males are significantly negatively correlated with FSH and have a negative feedback effect on FSH. Shortly after the birth of the male, the serum inhibin B levels gradually increase, reaching the adult level in the second phase of puberty. There is a negative correlation between the inhibin B and FSH from the third phase of puberty to adulthood. The inhibin B levels reach another peak from 20 years old to 30 years old, and after which the inhibin B levels gradually decrease with age. The main role of the inhibin B is to regulate gamete development through negative feedback to FSH. The inhibin B can be used as an endocrine label to monitor the gonad function of male or female by detecting it. Therefore, inhibin B is also very important in the detection of male infertility.

The inhibin B is a major label of ovarian reserve function and testicular seminiferous tubule function, and can be used for detection of female infertility caused by ovarian factors and male infertility caused by seminiferous tubule dysfunction, and can also be used to evaluate the diagnosis of cryptorchidism and precocious puberty in children, and to monitor the damage of male spermatogenic function by radiotherapy and chemotherapy.

The conventional methods of detecting the inhibin B include enzyme-linked immunosorbent assay and chemiluminescence assay, however, the conventional method of detecting the inhibin B has poor detection sensitivity.

### SUMMARY

Accordingly, it is necessary to provide an inhibin B chemiluminescence immunoassay kit with high detection sensitivity and a preparation method thereof.

An inhibin B chemiluminescence immunoassay kit includes a carboxylated magnetic particle coated with an inhibin B monoclonal antibody and an inhibin B monoclonal antibody labeled with a chemiluminescence label, wherein the inhibin B monoclonal antibody in each of the carboxylated magnetic particle coated with the inhibin B monoclonal antibody and the inhibin B monoclonal antibody labelled with the chemiluminescence label is the same.

A method of preparing the aforementioned inhibin B chemiluminescence immunoassay kit includes the steps of:

weighing a suspension of a carboxylated magnetic particle, removing supernatant by a magnetic separation, then resuspending with a MES (2-(N-morpholine)ethanesulfonic acid) buffer, and then adding an EDC (1-ethyl-3-(3-dimethyl aminopropyl)-carbodiimide) aqueous solution to activate a surface carboxyl group of the carboxylated magnetic particle, and then adding an inhibin B monoclonal antibody, resuspending at room temperature for 2 h to 10 h, and removing supernatant by the magnetic separation, and then resuspending with a Tris buffer to obtain a carboxylated magnetic particle coated with the inhibin B monoclonal antibody; and

weighing an inhibin B monoclonal antibody, adding a carbonate buffer and mixing uniformly, and then adding a chemiluminescence label and mixing uniformly, reacting in the dark at room temperature for 1 h to 2 h and then removing impurity to obtain an inhibin B monoclonal antibody labeled with the chemiluminescence label, wherein the inhibin B monoclonal antibody in each of the carboxylated magnetic particle coated with the inhibin B monoclonal antibody and the inhibin B monoclonal antibody labelled with the chemiluminescence label is the same.

This inhibin B chemiluminescence immunoassay kit is capable of performing the detection of inhibin B by using a fully automatic chemiluminescence immunoassay analyzer as a detection tool. The experiment shows this inhibin B chemiluminescence immunoassay kit has a detection sensitivity of 10 pg/mL, which is at least 10 times more sensitive than a conventional method of detecting inhibin B. This inhibin B chemiluminescence immunoassay kit has a higher detection precision.

### BRIEF DESCRIPTION OF THE DRAWINGS

FIG. 1 is a flowchart of a method of preparing an inhibin B chemiluminescence immunoassay kit according to an embodiment; and
FIG. 2 is a standard curve diagram of inhibin B obtained in Example 3.

### DETAILED DESCRIPTION OF THE EMBODIMENTS

The above objects, features and advantages of the present invention will become more apparent by describing in detail embodiments thereof with reference to the accompanying drawings. Numerous specific details are set forth in the description below in order to provide a thorough understanding of the invention.

An inhibin B chemiluminescence immunoassay kit includes a carboxylated magnetic particle coated with an inhibin B monoclonal antibody and an inhibin B monoclonal antibody labeled with a chemiluminescence label, wherein the inhibin B monoclonal antibody in each of the carboxylated magnetic particle coated with the inhibin B monoclonal antibody and the inhibin B monoclonal antibody labelled with the chemiluminescence label is the same.

Preferably, in the carboxylated magnetic particle coated with the inhibin B monoclonal antibody, a mass ratio of the inhibin B monoclonal antibody to the carboxylated magnetic particle is 1:25 to 1:35.

Preferably, in the inhibin B monoclonal antibody labeled with the chemiluminescence label, a mass ratio of the inhibin B monoclonal antibody to the chemiluminescence label is 50:1 to 50:10.

The inhibin B monoclonal antibodies used in the carboxylated magnetic particle coated with the inhibin B monoclonal antibody and the inhibin B monoclonal antibody labeled with the chemiluminescence label are the same.

Preferably, the carboxylated magnetic particle has a particle size of 0.05 µm to 1 µm.

The chemiluminescence label can be luminol, isoluminol, terpyridine ruthenium, or acridinium ester. The chemiluminescence label is preferably an acridinium ester.

In alternative embodiments, the aforementioned inhibin B chemiluminescence immunoassay kit further includes a chemiluminescence substrate solution.

The chemiluminescence substrate solution includes a solution A and a solution B. The solution A can be a H₂O₂ solution, and the solution B can be a NaOH solution.

In the present embodiment, the solution A is a H₂O₂ solution with a concentration of 0.1 mol/L, and the solution B is a NaOH solution with a concentration of 0.25 mol/L.

In alternative embodiments, the aforementioned inhibin B chemiluminescence immunoassay kit further includes an inhibin B calibrator.

The inhibin B calibrator is a solution of inhibin B at concentrations of 0 pg/mL, 50 pg/mL, 200 pg/mL, 500 pg/mL, 1000 pg/mL, and 1600 pg/mL, respectively.

Specifically, the inhibin B calibrator may be prepared by formulating the inhibin B into the solution of inhibin B at concentrations of 0 pg/mL, 50 pg/mL, 200 pg/mL, 500 pg/mL, 1000 pg/mL, and 1600 pg/mL, respectively, using a calibration buffer.

When this inhibin B chemiluminescence immunoassay kit is used for the detection of inhibin B, the inhibin B calibrator is tested by the fully automatic chemiluminescence immunoassay analyzer, and a standard curve is drawn and established in computer software. Then, an actual sample is tested, and a concentration of the sample is calculated according to a luminescence value of the sample. Finally, the performances (sensitivity, linearity, precision, and interference) of the inhibin B fully automatic chemiluminescence immunoassay system are evaluated.

This inhibin B chemiluminescence immunoassay kit is capable of performing the detection of inhibin B by using a fully automatic chemiluminescence immunoassay analyzer as a detection tool. The experiment shows this inhibin B chemiluminescence immunoassay kit has a detection sensitivity of 10 pg/mL, which is at least 10 times more sensitive than a conventional method of detecting inhibin B. This inhibin B chemiluminescence immunoassay kit has a higher detection precision.

In addition, this inhibin B chemiluminescence immunoassay kit has the following advantages:

1. The acridinium ester is selected as a labeling material and applied in the chemiluminescence immunoassay system. The chemiluminescence system is a direct chemiluminescence, compared with a conventional enzymatic chemiluminescence, the reaction does not require a participation of enzymes, thus saving cost.

2. The chemiluminescence immunoassay system using the acridinium ester has a wide linearity range, which can reach 10 pg/mL to 1300 pg/mL, while the conventional inhibin B detection method has a linear range of 20 pg/mL to 1000 pg/mL.

3. The acridinium ester chemiluminescence immunoassay system has a high reproducibility, and the intra-assay and inter-assay differences are within 5%, which is difficult to achieve by other chemiluminescence immunoassay systems.

4. The chemiluminescence immunoassay system has achieved the quantification analysis of the sample. By establishing standard curve to the test software, the concentration value of the sample can be directly obtained by simply testing the sample.

5. The chemiluminescence immunoassay system can be fully automated, and the addition of reagents and samples is completed by instruments, which makes the operation easier and reduces human error.

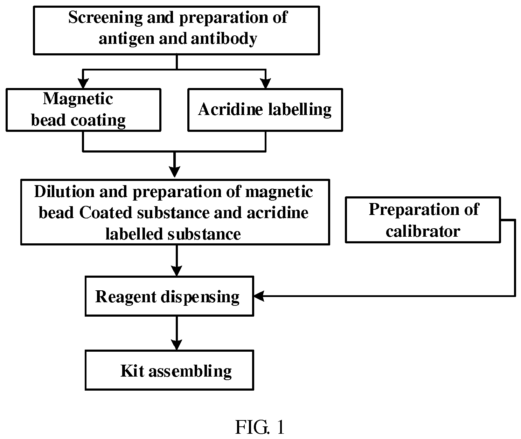

Referring to FIG. 1, a method of preparing the aforementioned inhibin B chemiluminescence immunoassay kit includes the steps of:

weighing a suspension of a carboxylated magnetic particle, removing supernatant by a magnetic separation, then resuspending with a MES (2-(N-morpholine)ethanesulfonic acid) buffer, and then adding an EDC (1-ethyl-3-(3-dimethyl aminopropyl)-carbodiimide) aqueous solution to activate a surface carboxyl group of the carboxylated magnetic particle, and then adding an inhibin B monoclonal antibody, resuspending at room temperature for 2 h to 10 h, and removing supernatant by the magnetic separation, and then resuspending with a Tris buffer to obtain a carboxylated magnetic particle coated with the inhibin B monoclonal antibody; and

weighing an inhibin B monoclonal antibody, adding a carbonate buffer and mixing uniformly, and then adding a chemiluminescence label and mixing uniformly, reacting in the dark at room temperature for 1 h to 2 h and then removing impurity to obtain an inhibin B monoclonal antibody labeled with the chemiluminescence label, wherein the inhibin B monoclonal antibody in each of the carboxylated magnetic particle coated with the inhibin B monoclonal antibody and the inhibin B monoclonal antibody labelled with the chemiluminescence label is the same.

MES (2-(N-morpholine)ethanesulfonic acid) buffer has a concentration of 0.02 M and a pH of 5.5.

Tris buffer has a concentration of 0.1 M and contains 2% of BSA, and has a pH of 8.0.

EDC (1-ethyl-3-(3-dimethyl aminopropyl)-carbodiimide) aqueous solution has a concentration of 10 mg/mL to 20 mg/mL, and a mass ratio of EDC to the carboxylated magnetic particle ranges from 0.05: 0.1 to 0.05:1.

Preferably, in the carboxylated magnetic particle coated with the inhibin B monoclonal antibody, a mass ratio of the inhibin B monoclonal antibody to the carboxylated magnetic particle is 1:25 to 1:35.

Preferably, the carboxylated magnetic particle has a particle size of 0.05 µm to 1 µm.

The carbonate buffer has a concentration of 0.1M and a pH of 9.0 to 9.5.

The impurity removal operation is desalination by a centrifugal desalting column. The specific operation is as follows: the centrifugal desalting column is firstly treated with purified water and TBS buffer (40 mM Tris-HCl, 0.5% BSA, 1% NaCl, pH 8.0), respectively; and finally the obtained solution of the carboxylated magnetic particle coated with the inhibin B monoclonal antibody is added, and finally the liquid in the centrifuge tube is collected.

Preferably, in the inhibin B monoclonal antibody labeled with the chemiluminescence label, a mass ratio of the inhibin B monoclonal antibody to the chemiluminescence label is 50:1 to 50:10.

The chemiluminescence label can be luminol, isoluminol, terpyridine ruthenium, or acridinium ester. The chemiluminescence label is preferably an acridinium ester.

The aforementioned inhibin B chemiluminescence immunoassay kit can be obtained by combining the obtained carboxylated magnetic particle coated with the inhibin B monoclonal antibody and the inhibin B monoclonal antibody labeled with the chemiluminescence label.

This inhibin B chemiluminescence immunoassay kit further requires a chemiluminescence substrate solution and an inhibin B calibrator when used.

The chemiluminescence substrate solution and the inhibin B calibrator can be prepared by themselves.

The chemiluminescence substrate solution includes a solution A and a solution B. The solution A can be a H₂O₂ solution, and the solution B can be a NaOH solution.

In the present embodiment, the solution A is a H₂O₂ solution with a concentration of 0.1 mol/L, and the solution B is a NaOH solution with a concentration of 0.25 mol/L.

Specifically, the inhibin B calibrator may be prepared by formulating the inhibin B into the solution of inhibin B at concentrations of 0 pg/mL, 50 pg/mL, 200 pg/mL, 500 pg/mL, 1000 pg/mL, and 1600 pg/mL, respectively, using a calibration buffer.

The preparation method of the inhibin B chemiluminescence immunoassay kit is simple and convenient, and the prepared inhibin B chemiluminescence immunoassay kit has high detection sensitivity and has a good application prospect.

The following are specific examples.

### Example 1: preparation of an inhibin B chemiluminescence immunoassay kit

### (1) Preparation of carboxylated magnetic particles coated with inhibin B monoclonal antibody:

A suspension containing 50 mg of carboxylated magnetic particles (MagnaBind^{™}, Cat. No. 21353) with a particle size of 0.05µm to 1µm was weighed, and magnetically separated to remove supernatant, and resuspended with 0.02 M of MES buffer with a pH of 5.5. 1 mL of newly prepared 10mg/mL EDC aqueous solution was added to activate the carboxyl group on the surface of the magnetic particles. 4 mg of inhibin B monoclonal antibody (biorbyt, Cat. No. orb48780) was added and suspended at room temperature for 6 h, and magnetically separated to remove supernatant. 0.1 M of Tris buffer containing 2% of BSA with a pH of 8.0 was used to resuspend to 1 mg/mL, thereby obtaining the carboxylated magnetic particles coated with the inhibin B monoclonal antibody, which was dispensed and stored in a 5 mL portions of each bottle at a temperature of 4 °C for use.

### (2) Preparation of acridinium ester labeled with inhibin B monoclonal antibody:

50 µL of inhibin B monoclonal labeled antibody (biorbyt, Cat. No. orb48780) solution with a concentration of 25mg/mL was weighed, 150 µL of carbonate buffer with a concentration of 0.1 M and a pH of 9.0 to 9.5 was added and mixed uniformly, and then 1.5 µL of acridinium ester with a concentration of 5 mg/mL was added and mixed uniformly, and reacted at room temperature in the dark. The reaction mixture was taken out after 1.5 h and desalted by 2 mL of zeba centrifugal desalting column. During the desalting process, the treatment was performed firstly by purified water and TBS buffer, respectively, and finally the obtained solution of the acridinium ester labeled with the inhibin B monoclonal antibody was added. The liquid in the centrifuge tube was collected to a preservation tube to obtain the acridinium ester labeled with the inhibin B monoclonal antibody, which was dispensed and stored in a 5 mL portions of each bottle at a temperature of 4 °C for use.

### (3) Preparation of inhibin B calibrator:

Inhibin B was formulated to concentrations of 0 pg/mL, 50 pg/mL, 200 pg/mL, 500 pg/mL, 1000 pg/mL, and 1600 pg/mL using calibrator buffer (40 mM Tris-HCl, 0.5% BSA, 1% NaCl, pH 8.0), which was dispensed in 0.5 mL per bottle and lyophilized, and stored at a temperature of 4 °C for use.

### Example 2: inhibin B chemiluminescence immunoassay method

A fully automatic chemiluminescence immunoassay analyzer (YHLO, Cat. No. iFlash3000) was used as a detection tool. The methodological mode was a double antibody sandwich method, that is, 50 µL of sample, 50 µL of carboxylated magnetic particles coated with the inhibin B monoclonal antibody, and 50 µL of inhibin B treatment solution were sequentially added by the instrument. 50 µL of acridinium ester coated with inhibin B was added after reacted for 20 min, and then magnetic separation was performed after reacted for 20 min. The instrument fed the reaction mixture into a darkroom, and sequentially added a luminescence substrate solution A (H₂O₂) and solution B (NaOH) to perform the luminescence reaction. Finally, the luminescence intensity was recorded.

### Example 3: performance evaluation of the inhibin B chemiluminescence immunoassay kit

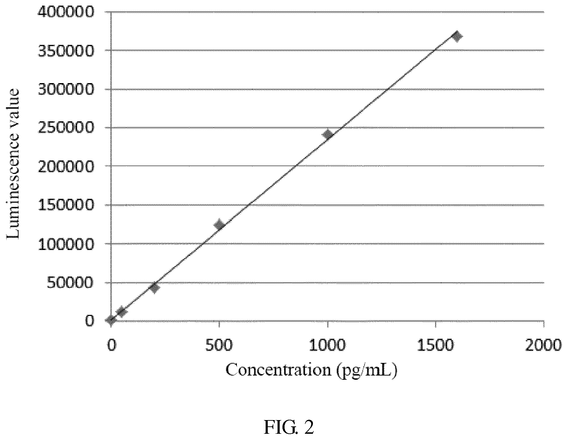

The inhibin B calibrator was tested by using the method in Example 2, and a standard curve was drawn as shown in FIG. 2.

Subsequently, an actual sample was next tested, and a concentration of the sample was calculated according to a luminescence value of the sample.

### Sensitivity test:

The sensitivity of the inhibin B chemiluminescence immunoassay kit was calculated with reference to the experimental protocol recommended in the CLSI EP17-A document, and the obtained sensitivity was 10 pg/mL.

### Linearity test:

Linear analysis was performed on the calibrators at concentrations of 50 pg/mL, 200 pg/mL, 500 pg/mL, 1000 pg/mL, and 1600 pg/mL, and the linear correlation coefficient was calculated, r=0.9996. In addition, the kit has a linear range of 10 pg/ml to 1300 pg/mL for detection of the inhibin B samples.

### Precision measurement:

Two inhibin B samples at concentrations of 500 pg/mL and 1000 pg/mL were weighed. Each of the samples was performed for 3 parallels for each concentration and the detection was performed with three batches of kits. The intra-assay and inter-assay differences of the kit were calculated, and the results showed that the intra-assay and inter-assay differences of that kit were both less than 5%.

### Interference experiment:

The pooled serum was weighed, and interfering substances including: conjugated bilirubin, free bilirubin, hemoglobin, ascorbic acid, and glyceride were added, respectively. The adding mass ratio was in accordance with 1:20. The measured values of the pooled serum and the pooled serum after the addition of various interfering substances were measured, respectively, and the deviation between the two was calculated, and the range of ±10% was taken as an acceptable range. The results showed that the interference had reached the NCCLS document standard and can be used for accurate assessment of adiponectin status in clinical laboratories.

### Example 4: comparative experiment of inhibin B chemiluminescence immunoassay kit

The inhibin B samples at concentrations of 50 pg/mL, 200 pg/mL, 500 pg/mL, 1000 pg/mL, and 1600 pg/mL were tested by the chemiluminescence detection method and the conventional enzyme linked immunosorbent assay, respectively. The detection sensitivities of the two methods were compared, and the results were shown in the following table:

| Number of tests | | Chemiluminescence detection (RLU) | Enzyme linked immunosorbent assay (OD) |
|---|---|---|---|
| 1 | | 1057 | 0.066 |
| 2 | | 1206 | 0.079 |
| 3 | | 1097 | 0.069 |
| 4 | | 1063 | 0.081 |
| 5 | | 1110 | 0.073 |
| 6 | | 1017 | 0.069 |
| 7 | | 1047 | 0.056 |
| 8 | | 1107 | 0.074 |
| 9 | | 989 | 0.063 |
| 10 | | 1039 | 0.059 |
| 11 | | 1102 | 0.087 |
| 12 | | 1053 | 0.068 |
| 13 | | 1004 | 0.074 |
| 14 | | 1058 | 0.068 |
| 15 | | 1110 | 0.071 |
| 16 | | 1055 | 0.072 |
| 17 | | 1048 | 0.067 |
| 18 | | 1102 | 0.081 |
| 19 | | 992 | 0.067 |
| 20 | | 1080 | 0.069 |
| Mean | | 1067 | 0.071 |
| SD | | 50 | 0.007 |
| M+2SD | | 1168 | 0.086 |
| 50pg/mL | 1 | 11206 | 0.133 |
| | 2 | 10936 | 0.141 |
| | 3 | 10498 | 0.142 |
| | Mean | 10880 | 0.139 |
| Sensitivity (pg/mL) | | 0.51 | 10.42 |

As can be seen from the above table, the sensitivity of the chemiluminescence detection method is about 20 times higher than that of the enzyme linked immunosorbent assay.

The foregoing descriptions are merely specific embodiments of the present invention.

The protection scope of the present invention shall be subject to the protection scope of the appended claims.

## Claims

1. An inhibin B chemiluminescence immunoassay kit, comprising a carboxylated magnetic particle coated with an inhibin B monoclonal antibody; and an inhibin B monoclonal antibody labeled with a chemiluminescence label, wherein the inhibin B monoclonal antibody in each of the carboxylated magnetic particle coated with the inhibin B monoclonal antibody and the inhibin B monoclonal antibody labelled with the chemiluminescence label is the same.

2. The inhibin B chemiluminescence immunoassay kit according to claim 1, wherein in the carboxylated magnetic particle coated with the inhibin B monoclonal antibody, a mass ratio of the inhibin B monoclonal antibody to the carboxylated magnetic particle is 1:25 to 1:35.

3. The inhibin B chemiluminescence immunoassay kit according to claim 1, wherein in the inhibin B monoclonal antibody labeled with the chemiluminescence label, a mass ratio of the inhibin B monoclonal antibody to the chemiluminescence label is 50:1 to 50:10.

4. The inhibin B chemiluminescence immunoassay kit according to claim 1, wherein the carboxylated magnetic particle has a particle size of 0.05 µm to 1 µm.

5. The inhibin B chemiluminescence immunoassay kit according to claim 1, wherein the chemiluminescence label is luminol, isoluminol, terpyridine ruthenium, or acridinium ester.

6. The inhibin B chemiluminescence immunoassay kit according to claim 1, further comprising a chemiluminescence substrate solution, wherein the chemiluminescence substrate solution comprises a solution A and a solution B, wherein the solution A is a H₂O₂ solution, and the solution B is a NaOH solution.

7. The inhibin B chemiluminescence immunoassay kit according to claim 1, further comprising an inhibin B calibrator.

8. The inhibin B chemiluminescence immunoassay kit according to claim 7, wherein the inhibin B calibrator is a solution of inhibin B at concentrations of 0 pg/mL, 50 pg/mL, 200 pg/mL, 500 pg/mL, 1000 pg/mL, and 1600 pg/mL, respectively.

9. A method of preparing an inhibin B chemiluminescence immunoassay kit according to any one of claims 1 to 8, comprising the steps of:
weighing a suspension of a carboxylated magnetic particle, removing supernatant by a magnetic separation, then resuspending with a MES (2-(N-morpholine)ethanesulfonic acid) buffer, and then adding an EDC (1-ethyl-3-(3-dimethyl aminopropyl)-carbodiimide) aqueous solution to activate a surface carboxyl group of the carboxylated magnetic particle, and then adding an inhibin B monoclonal antibody, resuspending at room temperature for 2 h to 10 h,
and removing supernatant by the magnetic separation, and then resuspending with a Tris buffer to obtain a carboxylated magnetic particle coated with the inhibin B monoclonal antibody; and
weighing an inhibin B monoclonal antibody, adding a carbonate buffer and mixing uniformly, and then adding a chemiluminescence label and mixing uniformly, reacting in the dark at room temperature for 1 h to 2 h and then removing impurity to obtain an inhibin B monoclonal antibody labeled with the chemiluminescence label,
wherein the inhibin B monoclonal antibody in each of the carboxylated magnetic particle coated with the inhibin B monoclonal antibody and the inhibin B monoclonal antibody labelled with the chemiluminescence label is the same.

## Patentansprüche

1. Inhibin-B-Chemilumineszenz-Immunassayset, umfassend ein carboxyliertes magnetisches Teilchen, das mit einem monoklonalen Inhibin-B-Antikörper beschichtet ist; und einen monoklonalen Inhibin-B-Antikörper, der mit einer Chemilumineszenzmarkierung markiert ist, wobei der monoklonale Inhibin-B-Antikörper in jedem des carboxylierten magnetischen Teilchens, das mit dem monoklonalen Inhibin-B-Antikörper beschichtet ist, und der monoklonale Inhibin-B-Antikörper, der mit der Chemilumineszenzmarkierung markiert ist, derselbe ist.

2. Inhibin-B-Chemilumineszenz-Immunassayset nach Anspruch 1, wobei in dem carboxylierten magnetischen Teilchen, das mit dem monoklonalen Inhibin-B-Antikörper beschichtet ist, ein Massenverhältnis des monoklonalen Inhibin-B-Antikörpers zu dem carboxylierten magnetischen Teilchen 1:25 bis 1:35 beträgt.

3. Inhibin-B-Chemilumineszenz-Immunassayset nach Anspruch 1, wobei in dem monoklonalen Inhibin-B-Antikörper, der mit der Chemilumineszenzmarkierung markiert ist, ein Massenverhältnis des monoklonalen Inhibin-B-Antikörpers zu der Chemilumineszenzmarkierung 50:1 bis 50:10 beträgt.

4. Inhibin-B-Chemilumineszenz-Immunassayset nach Anspruch 1, wobei das carbo-xylierte magnetische Teilchen eine Teilchengröße von 0,05 µm bis 1 µm aufweist.

5. Inhibin-B-Chemilumineszenz-Immunassayset nach Anspruch 1, wobei die Chemilumineszenzmarkierung Luminol, Isoluminol, Terpyridinruthenium oder Acridiniumester ist.

6. Inhibin-B-Chemilumineszenz-Immunassayset nach Anspruch 1, das weiters eine Chemilumineszenzsubstratlösung umfasst, wobei die Chemilumineszenzsubstratlösung eine Lösung A und eine Lösung B umfasst, wobei die Lösung A eine H₂O₂-Lösung ist und die Lösung B eine NaOH-Lösung ist.

7. Inhibin-B-Chemilumineszenz-Immunassayset nach Anspruch 1, das weiters einen Inhibin-B-Kalibrator umfasst.

8. Inhibin-B-Chemilumineszenz-Immunassayset nach Anspruch 7, wobei der Inhibin-B-Kalibrator eine Lösung von Inhibin B in einer Konzentration von 0 pg/ml, 50 pg/ml, 200 pg/ml, 500 pg/ml, 1000 pg/ml bzw. 1600 pg/ml ist.

9. Verfahren zur Herstellung eines Inhibin-B-Chemilumineszenz-Immunassaysets nach einem der Ansprüche 1 bis 8, das folgende Schritte umfasst:
Wiegen einer Suspension eines carboxylierten magnetischen Teilchens, Entfernen des Überstands durch Magnettrennung, dann Resuspendieren mit einem MES- (2-(N-Morpholin)ethansulfonsäure-) Puffer und dann Zusetzen einer wässrigen EDC- (1-Ethyl-3-(3-dimethylaminopropyl)carbodiimid-) Lösung zum Aktivieren einer Oberflächencarboxylgruppe des carboxylierten magnetischen Teilchens und dann Zusetzen eines monoklonalen Inhibin-B-Antikörpers, Resuspendieren bei Raumtemperatur für 2 h bis 10 h und Entfernen des Überstands durch Magnettrennung und dann Resuspendieren mit einem Tris-Puffer, um ein carboxyliertes magnetisches Teilchen zu erhalten, das mit dem monoklonalen Inhibin-B-Antikörper beschichtet ist; und
Wiegen eines monoklonalen Inhibin-B-Antikörpers, Zusetzen eines Carbonatpuffers und gleichmäßiges Vermischen und dann Zusetzen einer Chemilumineszenzmarkierung und gleichmäßiges Vermischen, Umsetzen im Dunkeln bei Raumtemperatur für 1 h bis 2 h und dann Entfernen von Verunreinigungen, um einen monoklonalen Inhibin-B-Antikörper zu erhalten, der mit einer Chemilumineszenzmarkierung markiert ist,
wobei der monoklonale Inhibin-B-Antikörper in jedem des carboxylierten magnetischen Teilchens, das mit dem monoklonalen Inhibin-B-Antikörper beschichtet ist, und der monoklonale Inhibin-B-Antikörper, der mit der Chemilumineszenzmarkierung markiert ist, derselbe ist.

## Revendications

1. Kit de dosage immunologique chimioluminescent d'inhibine B, comprenant des particules magnétiques carboxylées revêtues d'un anticorps monoclonal d'inhibine B ; et un anticorps monoclonal d'inhibine B marqué par un marqueur chimioluminescent, dans lequel l'anticorps monoclonal d'inhibine B dans chacune des particules magnétiques carboxylées revêtues de l'anticorps monoclonal d'inhibine B et l'anticorps monoclonal d'inhibine B marqué par un marqueur chimioluminescent sont identiques.

2. Kit de dosage immunologique chimioluminescent d'inhibine B selon la revendication 1, dans lequel dans les particules magnétiques carboxylée revêtues de l'anticorps monoclonal d'inhibine B, un rapport de masse de l'anticorps monoclonal d'inhibine B aux particules magnétiques carboxylées est de 1:25 à 1:35.

3. Kit de dosage immunologique chimioluminescent d'inhibine B selon la revendication 1, dans lequel, dans l'anticorps monoclonal d'inhibine B marqué par le marqueur chimioluminescent, un rapport de masse de l'anticorps monoclonal d'inhibine B au marqueur chimioluminescent est de 50:1 à 50:10.

4. Kit de dosage immunologique chimioluminescent d'inhibine B selon la revendication 1, dans lequel les particules magnétiques carboxylées ont une taille de particule de 0,05 µm à 1 µm.

5. Kit de dosage immunologique chimioluminescent d'inhibine B selon la revendication 1, dans lequel le marqueur chimioluminescent est le luminol, l'isoluminol, un terpyridine-ruthénium ou un ester d'acridinium.

6. Kit de dosage immunologique chimioluminescent d'inhibine B selon la revendication 1, comprenant en outre une solution de substrat de chimioluminescence, dans lequel la solution de substrat de chimioluminescence comprend une solution A et une solution B, dans lequel la solution A est une solution de H₂O₂, et la solution B est une solution de NaOH.

7. Kit de dosage immunologique chimioluminescent d'inhibine B selon la revendication 1, comprenant en outre un étalonneur d'inhibine B.

8. Kit de dosage immunologique chimioluminescent d'inhibine B selon la revendication 7, dans lequel l'étalonneur d'inhibine B est une solution d'inhibine B à des concentrations de 0 pg/ml, 50 pg/ml, 200 pg/ml, 500 pg/ml, 1 000 pg/ml et 1 600 pg/ml, respectivement.

9. Procédé de préparation d'un kit de dosage immunologique chimioluminescent d'inhibine B selon l'une quelconque des revendications 1 à 8, comprenant les étapes consistant à :
peser une suspension de particules magnétiques carboxylées,
éliminer le surnageant par une séparation magnétique, puis remettre en suspension avec un tampon MES (acide 2-(N-morpholine)éthanesulfonique), puis ajouter une solution aqueuse EDC (1-éthyl-3-(3-diméthylaminopropyl)-carbodiimide) pour activer un groupe carboxyle de surface de la particule magnétique carboxylée, puis ajouter un anticorps monoclonal d'inhibine B, remettre en suspension à température ambiante pendant 2 h à 10 h, et éliminer le surnageant par la séparation magnétique, puis remettre en suspension avec un tampon Tris pour obtenir des particules magnétiques carboxylées revêtues d'anticorps monoclonal d'inhibine B ; et
peser un anticorps monoclonal d'inhibine B, ajouter un tampon de carbonate et mélanger uniformément, puis ajouter un marqueur chimioluminescent et mélanger uniformément, faire réagir dans l'obscurité à température ambiante pendant 1 h à 2 h, puis éliminer l'impureté pour obtenir un anticorps monoclonal d'inhibine B marqué par le marqueur chimioluminescent,
dans lequel l'anticorps monoclonal d'inhibine B dans chacune des particules magnétiques carboxylées revêtues de l'anticorps monoclonal d'inhibine B et l'anticorps monoclonal d'inhibine B marqué par le marqueur chimioluminescent sont identiques.
